Europäisches Patentamt

European Patent Office

Office européen des brevets

(11) Publication number: **0 179 869 B1**

# EUROPEAN PATENT SPECIFICATION

(45) Date of publication of patent specification: **21.10.92**

(51) Int. Cl.5: **B01J 21/00**, B01J 23/04, C07C 2/00

(21) Application number: **85902301.2**

(22) Date of filing: **15.04.85**

(86) International application number: **PCT/US85/00687**

(87) International publication number: **WO 85/04821 (07.11.85 85/24)**

(54) **Supported catalysts and their use for converting methane and for dehydrogenating hydrocarbons.**

(30) Priority: **16.04.84 US 600912**
**16.04.84 US 600925**
**16.04.84 US 600926**
**16.04.84 US 600940**
**16.04.84 US 601136**
**18.12.84 US 683118**

(43) Date of publication of application:
**07.05.86 Bulletin 86/19**

(45) Publication of the grant of the patent:
**21.10.92 Bulletin 92/43**

(84) Designated Contracting States:
**AT BE CH DE FR GB IT LI LU NL SE**

(73) Proprietor: **ATLANTIC RICHFIELD COMPANY**
**515 South Flower Street**
**Los Angeles California 90071(US)**

(72) Inventor: **JAECKER, John, A.**
**2352 Clyde Terrace**
**Homewood, IL 60430(US)**
Inventor: **JONES, Andrew, C.**
**24 Clearbrook Road**
**Newtown Square, PA 19073(US)**
Inventor: **SOFRANKO, John, A.**
**Box 197, Line Road, RD4**
**Malvern, PA 19355(US)**
Inventor: **JOHNSON, Marvin, F., L.**
**1124 Elder Road**
**Homewood, IL 60430(US)**
Inventor: **WITHERS, Howard, P., Jr.**
**316 Laurelwood Drive**
**Douglasville, PA 19518(US)**
Inventor: **LEONARD, John, J.**
**31 Fairview Road**
**Springfield, PA 19064(US)**

Rank Xerox (UK) Business Services

EP 0 179 869 B1

(56) References cited:

| | |
|---|---|
| **US-A- 3 494 972** | **US-A- 3 642 930** |
| **US-A- 3 668 148** | **US-A- 3 804 902** |
| **US-A- 3 838 069** | **US-A- 3 928 238** |
| **US-A- 3 974 229** | **US-A- 3 987 104** |
| **US-A- 4 021 371** | **US-A- 4 205 194** |
| **US-A- 4 239 658** | **US-A- 4 397 771** |
| **US-A- 4 443 645** | **US-A- 4 443 648** |
| **US-A- 4 499 322** | |

**Journal of Catalysis, issued 1982, G.E. Keller and M.M. Bhasin, Synthesis of Ethylene via Oxidative Coupling of Methane, see pages 9-18**

**Journal of the Chinese Chemical Society, Trelant Fang and Chuin-Teh Yeh, Catalytic Pyrolysis of Methane, see pages 265-273**

Inventor: **BREDER, William, E., Jr.**
**16153 Debra Drive**
**Oak Forest, IL 60452(US)**

(74) Representative: **Cropp, John Anthony David**
**MATHYS & SOUIRE 10 Fleet Street**
**London, EC4Y 1AY(GB)**

**Description**

BACKGROUND OF THE INVENTION

Field of the Invention

This invention relates to a novel catalyst composition for the synthesis of hydrocarbons from a methane source and for the dehydrogenation of dehydrogenatable hydrocarbon. A particular application of this invention is a method for converting natural gas to more readily transportable material.

Description of the Pertinent Art

A major source of methane is natural gas. Other sources of methane have been considered for fuel supply (e.g., the methane present in coal deposits or formed during mining operations). Relatively small amounts of methane are also produced in various petroleum processes.

The composition of natural gas at the wellhead varies, but the major hydrocarbon present is methane. For example, the methane content of natural gas may vary within the range of about 40 to about 95 volume percent. Other constituents of natural gas include ethane, propane, butane, pentane (and heavier hydrocarbons), hydrogen sulfide, carbon dioxide, helium, and nitrogen.

Natural gas is classified as dry or wet, depending upon the amount of condensable hydrocarbons contained in it. Condensable hydrocarbons generally comprise $C_3 +$ hydrocarbons, although some ethane may be included. Gas conditioning is required to alter the composition of wellhead gas; processing facilities usually being located in or near the production fields. Conventional processing of wellhead natural gas yields processed natural gas containing at least a major amount of methane.

Large-scale use of natural gas often requires a sophisticated and extensive pipeline system. Liquefaction has also been employed as a transportation means, but processes for liquefying, transporting and revaporizing natural gas are complex, energy intensive and require extensive safety precautions. Transport of natural gas has been a continuing problem in the exploitation of natural gas resources. It would be extremely valuable to be able to convert methane (e.g., natural gas) to more readily transportable products. Moreover, direct conversion to olefins such as ethylene or propylene would be extremely valuable to the chemical industry.

Recently, it has been discovered that methane may be converted to higher hydrocarbons by a process which comprises contacting methane with an oxidative synthesizing agent at synthesizing conditions (e.g., at a temperature selected within the range of about 500° to about 1000°C). An oxidative synthesizing agent is a composition having as a principal component at least one oxide of at least one metal, which composition produces higher (i.e. $C_2 +$) hydrocarbon products, water and a composition comprising a reduced metal oxide when contacted with methane at synthesizing conditions. Reducible oxides of several metals have been identified which are capable of converting methane to higher hydrocarbons. In particular, oxides of manganese, tin, indium, germanium, lead, antimony and bismuth are most useful.

The Journal of Catalysis 73 (1982) pages 9 to 19 describes the synthesis of ethylene and ethane by catalytic oxidative coupling of methane at atmospheric pressure and temperatures in the range 500 to 1000°C and in the presence of a metal oxide supported on α-alumina. The best result recorded, which employs an oxide of manganese, gives a yield of 5% of $C_2$ hydrocarbons.

The reaction products of such processes are mainly ethylene, ethane, other light hydrocarbons, carbon oxides, coke and water. It would be beneficial to these oxidative synthesis processes to reduce the production of carbon oxides and coke.

Accordingly, an object of this invention is to provide an improved process for converting methane to higher hydrocarbons. A further object of this invention is an improved oxidative synthesizing agent - one capable of converting methane with reduced byproduct selectivities.

Other aspects, objects and the several advantages of this invention will become apparent to those skilled in the art upon reading this Specification and the appended claims.

According to the present invention there is provided a catalyst composition for the conversion of methane to higher hydrocarbons, said composition comprising

(a) at least one reducible oxide of at least one metal, which oxide is reduced and produces higher hydrocarbon products and water when contacted with methane at a temperature in the range of 500°C to 1000°C;

(b) an alkali metal; and

(c) a support selected from one or more of (i) pre-calcined hydroxylated magnesia and (ii) mixed oxides

3

of silicon and at least one alkaline earth metal.

The invention also provides a method for the conversion of methane to produce hydrocarbons having a greater number of carbon atoms, said method comprising contacting methane with the above-mentioned catalyst composition under conditions to form said hydrocarbons, by-product water and a reduced form of said at least reducible oxide.

The catalyst composition of this invention, sometimes referred to herein as a contact agent, includes at least one reducible oxide of at least one metal and a support. The reducible oxide produces higher hydrocarbon products, water and a reduced metal oxide when contacted with methane at a temperature selected within the range of 500° to 1000°C. The term "reducible" is used to identify those oxides of metals which are reduced by contacting methane at synthesizing conditions. The term "oxide(s) of metal(s)" includes: (1) one or more metal oxides (i.e., compounds described by the general formula $M_xO_y$ wherein M is a metal, O is oxygen, and the subscripts x and y designate the relative atomic proportions of metal and oxide in the composition); and/or (2) one or more oxygen-containing metal compounds; provided that such oxides and compounds have the capability of performing to produce higher hydrocarbon products as set forth herein.

The preferred contact agents comprise reducible oxides of metals selected from a group consisting of manganese, tin, indium, germanium, antimony, lead, bismuth, and mixtures thereof. The particularly preferred contact agents comprise reducible oxides of manganese and mixtures of reducible oxides of manganese with other oxidative synthesizing agents.

In the preferred embodiment, in addition to manganese, other reducible oxides of metals may be included in the compositions of this invention. These other reducible oxides of metals include tin, indium, germanium, antimony, lead, bismuth, praseodymium, terbium, cerium, iron, and ruthenium. However, in certain embodiments of this invention, the compositions are characterized by the substantial absence of a catalytically effective amount of iron to distinguish known oxidative dehydrogenation catalysts based on the use of manganese ferrites.

One class of preferred compositions is characterized by the substantial absence of catalytically effective amounts of nickel and the noble metals (e.g., rhodium, palladium, silver, osmium, iridium, platinum, and gold) and compounds thereof to minimize the deleterious catalytic effects of such metals and compounds thereof. For example, at the conditions (e.g., temperatures) under which the present compositions are used, these metals tend to promote coke formation and oxides of these metals tend to promote the formation of combustion products ($CO_x$), rather than the desired hydrocarbons. The term "catalytically effective" is used to identify that quantity of nickel, the noble metals, and compounds thereof which, when present, substantially changes the distribution of products obtained when employing the composition of this invention.

The support employed in the compositions of the present invention is selected from precalcined hydroxylated magnesia, mixed oxides of silicon and at least one alkaline earth metal, and mixtures thereof. By "precalcined" we mean that the hydroxylated magnesia has been calcined prior to the addition of the material providing the reducible metal oxide.

"The preferred mole ratio of the alkaline earth oxide to the silica in the mixed oxide is 1:1 or greater, and more preferably 30:1 to 5:1. Where the alkaline earth oxide is magnesia, the preferred mole ratio is 50:1 to 1:1 and more preferably 30:1 to 5:1. Particularly good results are obtained when the ratio of alkaline earth oxide to silica is about 5:1."

A class of compositions within the scope of this invention comprises manganese-containing oxides, at least one alkali metal or compound thereof, and mixed oxides of silicon and alkaline earth metals, said composition satisfying the formula:

$$MnA_aB_bSi_cO_x$$

wherein A is at least one alkali metal, B is at least one alkaline earth metal, a is within the range of 0.01 to 10, b is within the range of 0.1 to 90, c is within the range of 1 to 90, the sum of b plus c is greater than 1, and x is the number of oxygen atoms required to satisfy the valence states of the other elements. When B is magnesium, b is preferably within the range of 0.6 to 10. When B is calcium, b is preferably within the range of 0.4 to 10. When B is barium, b is preferably within the range of 0.1 to 5.

While the relative amounts of alkaline earth metals and silicon in the composition are not believed to be narrowly critical, preferred $SiO_2$-MgO components have been identified. One component consists of silica-magnesia wherein the ratio c:b is within the range of 2-3:1. Another component consists of magnesia-silica wherein the ratio b:c is within the range of 5-30:1.

The composition may be prepared from silica and alkaline earth oxides such as MgO, CaO, SrO, and BaO. However, other sources of mixed oxides may also be employed (e.g., $MgSiO_4$, $MgSiO_3$, $Mg_2SiO_4$, $CaMg(SiO_3)_2$, and $Ca_2BaSi_3O_9$).

The term "hydroxylated magnesia" means a magnesia derived from magnesium hydroxide or a magnesium-containing component contacted with a hydroxyl-containing component. The hydroxylated magnesia is preferably derived from magnesium hydroxide (e.g., magnesia produced from sea water). One such suitable magnesia is commercially available from CRI Industries as MgO-700.

Alternatively, the hydroxylated magnesia may be derived from sources other than magnesium hydroxide such as a magnesium-containing component contacted with a hydroxyl-containing material (e.g., one or more compounds including hydroxyl groups). Such hydroxyl-containing material includes sodium hydroxide, potassium hydroxide, lithium hydroxide, slake lime, calcium hydroxide, and hydroxides of barium. One method of producing the present hydroxylated magnesia comprises contacting a magnesium-containing component with (a) water for an extended period or (b) boiling water. Any suitable magnesium-containing component may be employed to produce hydroxylated magnesia. Examples include magnesia, magnesium chloride, and magnesium salts.

An essential feature of the present invention is the precalcination of the hydroxylated magnesia support prior to the addition of the at least one metal. The hydroxylated magnesia is calcined prior to the addition of the at least one metal at an elevated temperature in an oxygen-containing gas. The particular precalcining temperature will vary, but preferably it will be between about 300° and about 1200°C.

The support is preferably prepared in powdered form, more preferably having a particle size ranging from about 20 to about 200 $\mu$m, and still more preferably about 100 $\mu$m. The support is dried to the extent that upon subsequent sintering the particles do not steam or explode. Preferably, the particles have a water content of less than about 1.0 weight percent water.

Preferably, the support is sintered to an elevated temperature (i.e., heated to a high temperature without melting the support) by exposure for a short time to a temperature high enough to cause at least partial fusion of the surface of the particles. This exposure can occur before or after the addition of the metal which forms at least one reducible oxide (e.g., derived from sodium or lithium permanganate, or mixtures of manganese and sodium, or lithium salts). The elevated (sintering) temperature varies with the composition of the material being sintered. In one preferred embodiment, the elevated temperature is equal to about 0.33 of the normal melting temperature of the material of the support.

Exposure to the high temperature may be accomplished by allowing the particles to briefly contact a flame or a hot surface. Alternatively, a laser or other electromagnetic radiation source with a limited depth of surface penetration of the support may be used. The degree of surface sintering can be controlled by the temperature of the flame or of the hot surface, by the intensity of the light, or by the length of time of exposure.

The particles should be removed from the heat source quickly so that the effect of the sintering is confined to the depth desired. Removal from the flame or hot surface can be accomplished by several means--by transporting the particles out of the region of the hot substance, by cooling the hot substance with another material, by contacting the particles with a heat sink to remove the heat absorbed from the hot substance, or by combinations of these and other methods. When a laser is used, its light can be diverted or adsorbed. Removal of heat by radiation or conduction is preferred.

The addition of steam or an inert gas (such as nitrogen) or a reactive gas (such as hydrogen chloride) is preferred to control the sintering process.

Preferred sintering temperatures for the support material are in the range of about 920°C to about 2800°C (about 1690° to about 5070°F) for magnesia and about 560°C to about 1710°C (about 1040° to about 3110°F) for silica. Sintering of the support may take place in a period of time in the range of about 0.5 minute to about 15 minutes or more, preferably in a period of time in the range of about 1 minute to about 10 minutes.

The method of the present invention preferably provides support compositions exhibiting a surface area ranging from 30 to 90 square meters per gram.

Preferably, in the catalyst composition of this invention the reducible oxide is an oxide of manganese. In general, the preferred compositions contain more than about 50 weight percent of the support, more preferably they contain more than about 60 weight percent of the support. Stated in another way, manganese is preferably present in an amount within the range of 1 to 40 weight percent based on the combined weight of the manganese and the support, more preferably within the range of 5 to 30 weight percent. The atomic ratio of alkali metal to manganese is preferably within the range of 0.01:1 to 10:1.

Other additives may also be incorporated into the composition of this invention. For example, addition of a phosphorus component has been found to enhance the stability of the composition. When used, phosphorus may be present up to an amount providing a phosphorus-to-manganese ratio of about 2:1. If phosphorus is employed, it is desirable to provide it during catalyst preparation in the form of phosphates of alkali metals (e.g., orthophosphates, metaphosphates, and pyrophosphates). Pyrophosphates are preferred. Sodium pyrophosphate is particularly preferred. Phosphorus can be provided in other forms though. Examples include orthophosphoric acid, ammonium phosphates, and ammonium hydrogenphosphates.

Further examples of other components which may be present in the compositions of this invention include halogen and chalcogen components. Such components may be added either during preparation of the catalyst or during use.

The contact agent of this invention contains at least one alkali metal or compound thereof. Sodium and/or compounds thereof are a particularly preferred alkali metal component. Except as noted elsewhere herein, the atomic ratio in which these materials are combined to form the contact agent is not narrowly critical. However, the preferred atomic ratio of the reducible oxide component (expressed as the metal, e.g., Mn) to the alkali metal component (expressed as the metal, e.g., Na) is within the range of 0.1:1 to 100:1, more preferably within the range of 0.3:1 to 10:1. The preferred mole ratio of silica to alkali metal is 50:1 to 1:1, and more preferably 0.5:1 to 10:1. Most preferably, the ratio is 1:1 to 3:1.

The alkali metal component may be added to the support before or during precipitation, coprecipitation, or impregnation of the reducible oxide and the support.

The support or agent may be contacted with a suitable alkali metal component which should not interfere with the support function, the reducible oxide function, or the process for combining the support with the reducible oxide. Preferably, the alkali metal component is a basic composition of the alkali metal. More preferably, the alkali metal component is selected from a group consisting of sodium hydroxide, sodium acetate, lithium hydroxide, lithium acetate, and mixtures thereof.

The contact agent can be prepared by any suitable method. Conventional methods such as precipitation, coprecipitation, impregnation, granulation, and spray drying can be used.

One suitable method of preparation of the contact agent includes the preparation of an aqueous slurry of magnesia and silica gel and mixing the slurry with a solution of reducible oxides.

In a second suitable method, a support is prepared and dried, then impregnated with the suitable metal compounds which include acetates, acetylacetonates, oxides, carbides, carbonates, hydroxides, formates, oxalates, nitrates, phosphates, sulfates, sulfides, tartrates, fluorides, chlorides, bromides or iodides of the metals.

After the mixing of the slurry with the solution or the impregnation, the resulting composite is dried in an oven to remove solvent and the dried solid is prepared for use by calcining at elevated temperatures in an oxygen-containing gas (e.g., air) prior to use in the process of this invention. Particular calcination temperatures will vary, depending upon the particular metal compound or compounds employed. Preferably, the air temperature is selected within the range of about 300° to about 1200°C.

In addition to methane, the preferred feedstock employed in the method of this invention may contain other hydrocarbon or non-hydrocarbon components, although the methane content should typically be within the range of about 40 to about 100 volume percent, preferably about 80 to about 100 volume percent, more preferably about 90 to about 100 volume percent.

Operating temperatures for contacting the methane with the contact agent are preferably selected within the range of about 500° to about 1000°C.; the particular temperature selected depending upon the particular reducible metal oxide(s) employed in the contact agent. For example, reducible oxides of certain metals may require operating temperatures below the upper part of the recited range to minimize sublimation or volatilization of the metals (or compounds thereof) during methane contact. Examples include, reducible oxides of indium, germanium and bismuth (operating temperatures will preferably not exceed about 850°C.).

Operating pressures for the methane contacting step are not critical to the presently claimed invention. However, both general system pressure and partial pressure of methane have been found to affect overall results. Preferred operating pressures are within the range of 0.101 to 3.03 MPa (1 to 30 atmospheres).

Contacting methane and a reducible metal oxide to form higher hydrocarbons from methane also produces reduced metal oxides and water. The exact nature of the reduced metal oxides is unknown, and so is referred to herein as "reduced metal oxides". Regeneration of a reducible metal oxide is readily accomplished by contacting such reduced materials with oxygen (e.g., an oxygen-containing gas such as air) at elevated temperatures, preferably at a temperature selected within the range of about 300° to about 1200°C.; the particular temperature selected depending on the metal(s) included in the contact agent.

In carrying out the present process, a single reactor apparatus containing a fixed bed of solids may be used with intermittent or pulsed flow of a first gas comprising methane followed by intermittent or pulsed flow of a second gas comprising oxygen (e.g., oxygen, oxygen diluted with an inert gas, or air, preferably air). The methane contacting step and the oxygen contacting step may also be performed in physically separate zones with solids recirculating between the two zones.

Thus, a suitable method for synthesizing hydrocarbons from a methane source comprises: (a) contacting a gas comprising methane and particles comprising a contact agent to form higher hydrocarbon products, water and reduced metal oxides; (b) removing particles comprising reduced metal oxides from the first zone and contacting the reduced particles in a second zone with an oxygen-containing gas to form particles comprising a contact agent; and (c) returning the particles produced in the second zone to the first zone. The steps are preferably repeated at least periodically, and more preferably the steps are continuous. In the more preferred embodiment, solids are continuously circulated between at least one methane contact zone and at least one oxygen contact zone.

Particles comprising a reducible metal oxide which are contacted with methane may be maintained as fluidized, ebullating, or entrained beds of solids. Preferably, methane is contacted with a fluidized bed of solids.

Similarly, particles comprising reduced metal oxides which are contacted with oxygen may be maintained as fluidized, ebullating, or entrained beds of solids. Preferably, oxygen is contacted with a fluidized bed of solids.

In the more preferred embodiment of the present invention, methane feedstock and particles comprising a contact agent are continuously introduced into a methane contact zone maintained at synthesizing conditions. Synthesizing conditions include the temperatures and pressures described above. Gaseous reaction products from the methane contact zone (separated from entrained solids) are further processed (e.g., passed through a fractionating system wherein the desired hydrocarbon products are separated from unconverted methane and combustion products). Unconverted methane may be recovered and recycled to the methane contact zone.

Particles comprising reduced metal oxides are contacted with oxygen in an oxygen contact zone for a time sufficient to oxidize at least a portion of the reduced metal oxides to produce a reducible metal oxide and to remove (i.e., combust) at least a portion of any carbonaceous deposit which may form on the particles in the methane contact zone. The conditions of the oxygen contact zone will preferably include a temperature selected within the range of about 300° to about 1200°C., pressures of up to about 3.03 MPa (30 atmospheres), and average particle contact time within the range of about 1 minute to about 120 minutes. Sufficient oxygen is preferably provided to oxidize all reduced metal oxides to produce a reducible metal oxide and to completely combust any carbonaceous deposit material deposited on the particles. At least a portion of the particles comprising the contact agent which are produced in the oxygen contact zone are returned to the methane contact zone.

Another more specific application for the compositions of this invention is the dehydrogenation of dehydrogenatable hydrocarbons. The process comprises contacting a gas comprising a dehydrogenatable hydrocarbon with a composition of this invention to produce dehydrogenated hydrocarbon products, water, and a composition comprising a reduced metal oxide. Dehydrogenatable hydrocarbons include a wide variety of hydrocarbons (e.g., $C_2+$ alkanes, cycloalkanes, olefins, alkylaromatics, etc.). The dehydrogenated product depends in part on the feedstock selected. For example, alkanes may be dehydrogenated to form olefins, diolefins, alkynes, etc., and olefins may be dehydrogenated to form diolefins, alkynes, etc. One preferred class of feedstock comprises $C_2$-$C_4$ alkanes. One preferred process embodiment comprises oxidative dehydrogenation of $C_2$-$C_5$ alkanes to form the corresponding mono-olefins.

Operating temperatures for such a process are generally within the range of about 500° to about 1000°C. Operating pressures are not narrowly critical. In general, the process is conducted within the parameters of the oxidative dehydrogenation art, but uses a novel catalyst.

The rate of solids withdrawal from the methane contact zone is desirably balanced with the rate of solids passing from the oxygen contact zone to the methane contact zone so as to maintain a substantially constant inventory of particles in the methane contact zone, thereby enabling steady-state operation of the synthesizing system.

The present invention is further illustrated by reference to the following Examples.

EXAMPLES

Methane contact runs were made at about atmospheric pressure in quartz tube reactors (12 mm. inside diameter) partially packed with 10 ml. of contact solids. The reactors were brought up to temperature under a flow of heated nitrogen which was switched to methane at the start of the run. Unless otherwise indicated, all methane contact runs described in the Examples had a duration of two minutes. At the end of each methane contact run, the reactor was flushed with nitrogen and the solids were regenerated under a flow of heated air (usually at 800°C. for 30 minutes). The reactor was then again flushed with nitrogen and the cycle repeated. Most of the results reported below are based on the cumulative samples collected after the contact solids were "equilibrated" (i.e., after the aberrant characteristics of the fresh contact solids had dissipated). This allows more meaningful comparison between the contact solids within the scope of the present invention and other contact solids. Three to six cycles of methane contact and regeneration are generally sufficient to equilibrate the contact solids.

Space velocities are reported as gas hourly space velocities (hr.$^{-1}$) (GHSV) and were 600 GHSV, except where indicated otherwise.

Example I

27.6 grams of $NaMnO_4 \cdot 3H_2O$ (Pfaltz and Bauer SO-5560) were dissolved in 400 ml. of water. 138 grams of magnesia (Fisher MgO, heavy, U.S.P.) were calcined for 16 hours at 800°C. The sodium permanganate solution and the calcined magnesia were slurried for 1 hour at 150°F (66°C). The product was dried for 2 hours at 230°F (110°C)., crushed and then screened through a 60-mesh sieve. The powder was then calcined for 16 hours at 800°C.

Example II

27.6 grams of $NaMnO_4 \cdot 3H_2O$ (Pfaltz and Bauer SO-5560) were dissolved in 300 ml. of water. 122 grams of magnesia (Fisher MgO, heavy, U.S.P.) were calcined for 16 hours at 550°C. The sodium permanganate solution, 100 grams of colloidal silica (Nalco 2326), and the calcined magnesia were slurried for 1 hour at 150°F (66°C). The product was dried for 2 hours at 230°F (110°C)., crushed and then screened through a 60-mesh sieve. The powder was then calcined for 16 hours at 800°C.

Comparative Example III

27.6 grams of $NaMnO_4 \cdot 3H_2O$ (Pfaltz and Bauer SO-5560) were dissolved in 525 ml. of water. The solution and 144 grams of magnesia (Catalyst Resources, Inc. MgO-700 precursor (without addition of silica)) were slurried for 1 hour at 150°F (66°C). The solid material was dried for 2 hours at 230°F (110°C)., crushed and then screened through a 60-mesh sieve. The powder was then calcined for 16 hours at 800°C.

Example IV

27.6 grams of $NaMnO_4 \cdot 3H_2O$ (Pfaltz and Bauer SO-5560) were dissolved in 300 ml. of water. The solution, 128 grams of magnesia (Catalyst Resources, Inc. MgO-700 precursor (without addition of silica)) and 100 grams of colloidal silica (Nalco 2326) were slurried for 1 hour at 150°F (66°C). The solid material was dried for 2 hours at 230°F (110°C)., crushed and then screened through a 60-mesh sieve. The powder was then calcined for 16 hours at 800°C.

Example V

534 grams of $NaMnO_4 \cdot 3H_2O$ (Pfaltz and Bauer SO-5560) were dissolved in 1500 ml. of water and diluted up to 1900 ml. 2620 grams of magnesia (Basic Chemical Co. Magox 95) were calcined for 16 hours at 550°C. The calcined magnesia was impregnated with the sodium permanganate solution. The product was dried for 2 hours at 230°F (110°C)., crushed and then screened through a 60-mesh sieve. The powder was then calcined for 16 hours at 800°C.

Example VI

27.6 grams of $NaMnO_4 \cdot 3H_2O$ (Pfaltz and Bauer SO-5560) were dissolved in 100 ml. of water. The sodium permanganate solution, 100 grams of colloidal silica 2326) and 307 grams of magnesia (Basic Chemical Co. Magox 95 precursor, mud) were slurried for 1 hour at 150°F (66°C). The product was dried for 2 hours at 230°F (110°C)., crushed and then screened through a 60-mesh sieve. The powder was then calcined for 16 hours at 800°C.

Comparative Example VII

27.6 grams of NaMnO$_4$-3H$_2$O (Pfaltz and Bauer SO-5560) were dissolved in 400 ml. of water. 138 grams of magnesia (Malinckrodt MgO, U.S.P.) were calcined for 16 hours at 800°C. The sodium permanganate solution and the calcined magnesia were slurried for 1 hour at 150°F (66°C). The product was dried for 2 hours at 230°F (110°C)., crushed and then screened through a 60-mesh sieve. The powder was then calcined for 16 hours at 800°C.

### Example VIII

27.6 grams of NaMnO$_4$-3H$_2$O (Pfaltz and Bauer SO-5560) were dissolved in 300 ml. of water. 122 grams of magnesia (Malinckrodt MgO, U.S.P.) were calcined for 16 hours at 550°C. The sodium permanganate solution, 100 grams of colloidal silica (Nalco 2326) and the calcined magnesia were slurried for 1 hour at 150°F (66°C). The product was dried for 2 hours at 230°F (110°C)., crushed and then screened through a 60-mesh sieve. The powder was then calcined for 16 hours at 800°C.

Table 1

| Atomic Ratio vs. Na | | | | | | | | |
|---|---|---|---|---|---|---|---|---|
| Ex. No. | Test No. | Na | Mn | Mg | Si | % Conv. | % Selec. | % C$_2$ Yield |
| I | 5511-09 | 1 | 1 | 24.0 | -- | 14.2 | 81.8 | 11.6 |
| II | 5508-20 | 1 | 1 | 21.0 | 1.8 | 20.5 | 65.6 | 13.4 |
| III | 5523-06 | 1 | 1 | 24.0 | -- | 14.2 | 11.2 | 1.6 |
| IV | 5526-09 | 1 | 1 | 21.0 | 1.8 | 20.7 | 58.9 | 12.2 |
| V | 5504-19 | 1 | 1 | 24.0 | -- | 13.0 | 52.6 | 6.8 |
| VI | 5524-01 | 1 | 1 | 21.0 | 1.8 | 19.0 | 48.7 | 9.3 |
| VII | 5515-01 | 1 | 1 | 24.0 | -- | 14.3 | 82.5 | 11.8 |
| VIII | 5501-08 | 1 | 1 | 21.0 | 1.8 | 15.3 | 59.1 | 9.0 |

The silica/magnesia support associated with the reducible oxide is shown to be more selective than its counterpart with the silica/magnesia support.

### Example IX

470 grams of NaMnO$_4$-3H$_2$O (Pfaltz and Bauer SO-5560) were dissolved in 1000 ml. of water and diluted to 2617 ml. 2380 grams of magnesia (Catalyst Resources, Inc. MgO-700 tabletted magnesia) were crushed and calcined for 16 hours at 550°C. The calcined magnesia was impregnated with the sodium permanganate solution. The product was dried for 2 hours at 230°F (110°C)., and then calcined for 16 hours at 800°C.

### Example X

27.6 grams of NaMnO$_4$-3H$_2$O (Pfaltz and Bauer SO-5560) were dissolved in 100 ml. of water and diluted to 130 ml. 138 grams of magnesia (Fisher MgO, heavy, U.S.P.) were calcined for 16 hours at 550°C. The calcined magnesia was impregnated with the sodium permanganate solution. The product was dried for 2 hours at 230°F (110°C). and then calcined for 16 hours at 800°C.

### Example XI

27.6 grams of NaMnO$_4$-3H$_2$O (Pfaltz and Bauer SO-5560) were dissolved in 100 ml. of water and diluted to 130 ml. 138 grams of magnesia (Martin-Marietta 500 G30) were calcined for 16 hours at 550°C. The sodium permanganate solution was impregnated onto the calcined magnesia. The product was dried for 2 hours at 230°F (110°C). and then calcined for 16 hours at 800°C.

### Example XII

534 grams of NaMnO$_4$-3H$_2$O (Pfaltz and Bauer SO-5560) were dissolved in 1500 ml. of water and diluted to 1900 ml. 2620 grams of magnesia (Basic Chemical Co. Magox 95) were calcined for 16 hours at 550°C. The calcined magnesia was impregnated with the sodium permanganate solution. The product was dried for 2 hours at 230°F (110°C)., crushed and then screened through a 60-mesh sieve. The powder was then calcined for 16 hours at 800°C.

Results reported below in Table 2 are based on the analysis of cumulative samples collected during the third two-minute methane contact run for each contact agent. Run conditions were 800°C.

Table 2

| Example No. | Test No. | Cycle | % Conv. | % Selec. | % C$_2$ Yield |
|---|---|---|---|---|---|
| IX | 5511-08 | 3 | 15.1 | 67.1 | 10.1 |
| X | 5517-09 | 3 | 14.2 | 81.8 | 11.6 |
| XI | 5473-01 | 3 | 23.3 | 6.5 | 1.5 |
| XII | 5504-19 | 3 | 13:0 | 52.6 | 6.8 |

Example XIII

Magnesium chloride was dissolved in water and hydrolyzed with aqueous sodium hydroxide. The magnesium hydroxide precipitate that formed was collected by filtration, washed with water, dried at 110°C. and then calcined at 500°C. in air for 16 hours. This calcined magnesium hydroxide support was impregnated with aqueous sodium permanganate to a 13 percent loading of the permanganate and then calcined at 1000°C. in air for 16 hours. The contact agent composition contained 5 weight percent manganese and 2.1 weight percent sodium on magnesia. The contact agent was run in the methane conversion process to yield the results shown in Table 3 below.

Table 3

| °C | GHSV hr$^{-1}$ | % Conv | Selectivity | | | | | | |
|---|---|---|---|---|---|---|---|---|---|
| | | | C$_{2=}$ | C$_2$ | C$_3$ | C$_{4-7}$ | CO | CO$_2$ | Coke |
| 825 | 1200 | 23.8 | 33.1 | 15.0 | 5.8 | 4.3 | 1.5 | 40.2 | 0 |
| 850 | 1200 | 28.2 | 28.4 | 12.6 | 4.9 | 3.5 | 4.3 | 46.2 | 0 |
| 825 | 600 | 42.5 | 26.6 | 9.2 | 4.7 | 14.9 | 0 | 44.4 | 0 |

Comparative Example XIV

Magnesium chloride was dissolved in water and hydrolyzed with aqueous sodium hydroxide. The magnesium hydroxide precipitate that formed was collected by filtration, washed with water and dried at 110°C. This dried magnesium hydroxide cake was impregnated with aqueous sodium permanganate to a 13 percent loading of the permanganate and calcined at 1000°C. in air for 16 hours. The contact agent composition contained 5 weight percent manganese and 2.1 weight percent sodium on magnesia. The contact agent was run in the methane conversion process to yield the results shown in Table 4 below.

Table 4

| °C | GHSV hr$^{-1}$ | % Conv | Selectivity | | | | | | |
|---|---|---|---|---|---|---|---|---|---|
| | | | C$_{2=}$ | C$_2$ | C$_3$ | C$_{4-7}$ | CO | CO$_2$ | Coke |
| 825 | 1200 | 23.1 | 11.5 | 12.7 | 1.7 | .7 | 0 | 70.4 | 2.9 |
| 825 | 600 | 36.5 | 14.1 | 10.0 | 2.2 | .7 | 0 | 72.9 | 6.6 |

Example XV

The procedure of Example XIII was repeated, except that magnesium acetate was substituted for magnesium chloride. The results of the methane runs are shown in Table 5 below.

Table 5

| °C | GHSV hr$^{-1}$ | % Conv | Selectivity | | | | | | |
|---|---|---|---|---|---|---|---|---|---|
| | | | $C_{2=}$ | $C_2$ | $C_3$ | $C_{4-7}$ | CO | $CO_2$ | Coke |
| 825 | 1200 | 10.5 | 38.0 | 38.0 | 5.7 | 3.8 | 0 | 13.6 | .8 |
| 850 | 1200 | 11.6 | 34.4 | 28.0 | 5.2 | 2.9 | 0 | 27.9 | 1.6 |
| 825 | 600 | 16.7 | 43.5 | 34.0 | 6.3 | 4.2 | 0 | 11.4 | .6 |

Example XVI

Magnesium acetate was calcined at 450°C. in air for 16 hours to yield magnesium oxide. This calcined magnesium oxide was impregnated with aqueous sodium permanganate to a 13 percent loading of the permanganate and calcined at 1000°C. in air for 16 hours. The contact agent composition contained 5 weight percent manganese and 2.1 weight percent sodium on magnesia. The contact agent was run in the methane conversion process to yield the results shown in Table 6 below.

Table 6

| Temp °C | GHSV, hr$^{-1}$ | % Conversion | % $C_2$ Selectivity |
|---|---|---|---|
| 800 | 600 | 32.5 | 27.5 |
| 825 | 600 | 36.0 | 21.0 |
| 825 | 1200 | 22.0 | 18.0 |

**Claims**

1. A catalyst comprising (a) at least one compound, selected from oxides and oxygen-containing compounds of at least one metal, which compound is reducible upon contact with methane at a temperature in the range of 500° to 1000°C to produce higher hydrocarbons and water, and (b) a support; characterised in that the composition further includes (c) at least one alkali metal or compound thereof, and said support is selected from at least one of (i) hydroxylated magnesia which has been calcined prior to the addition of material providing said at least one compound of (a) and (ii) mixed oxides of silicon and at least one alkaline earth metal.

2. A composition as claimed in claim 1 characterised in that hydroxylated magnesia is derived from magnesium hydroxide or a magnesium-containing component contacted with a hydroxyl-containing material.

3. The composition of claim 1 characterised in that said support comprises magnesia and silica, in a mole ratio of magnesia to silica of 50:1 to 1:1, more preferably 30:1 to 5:1.

4. The composition of claim 1 or claim 3 characterised in that the mole ratio of said silica to said alkali metal is in the range of 0.5:1 to 10:1.

5. A composition as claimed in any one of claims 1 to 4 characterised in that the surface of at least a portion of said support is sintered at an elevated temperature prior to the addition of said at least one compound.

6. A composition as claimed in claim 5 characterised in that said sintering comprises heating said support to about 0.33 of the normal melting temperature of the material of said support.

11

**7.** A composition as claimed in claim 5 or claim 6 characterised in that the surface area of said support ranges from 30 to 90 square meters per gram.

**8.** The composition of any one of claims 1 to 7 characterised in that said at least one metal of compound (a) is selected from manganese, tin, indium, germanium, antimony, lead, bismuth, and mixtures thereof; and said alkali metal is selected from lithium, sodium, potassium, rubidum, cesium, and mixtures thereof, preferably sodium.

**9.** A composition as claimed in any one of claims 1 to 8 characterised in that said at least one metal of compound (a) comprises manganese and wherein the amount of said manganese present is within the range of 1 to 40 weight percent, and preferably from 5 to 30 weight percent, based upon the combined weight of said manganese and said support, said composition being substantially free of catalytically effective iron.

**10.** A composition as claimed in claim 9 characterised in that the atomic ratio of said alkali metal to said manganese is within the range of 0.01:1 to 10:1.

**11.** A composition as claimed in claim 9 or claim 10 characterised in that said composition satisfies the formula

$$MnA_aB_bSi_cO_x$$

where A is at least one alkali metal, B is at least one alkaline earth metal, a is within the range of 0.01 to 10, b is within the range of 0.1 to 90, c is within the range of 1 to 90, the sum of b + c is greater than 1 and x is the number of oxygen atoms required by the valence states of the other elements.

**12.** A composition as claimed in claim 11 characterised in that B is magnesium and b is preferably within the range of 0.6 to 10 or B is calcium and b is preferably within the range of 0.4 to 10, or B is barium and b is preferably within the range of 0.1 to 5.

**13.** A composition as claimed in claim 11 or claim 12 characterised in that B is magnesium and the ratio of c to b is within the range 2:1 to 3:1 or the ratio of b to c is within the range of 5:1 to 30:1.

**14.** A composition as claimed in claim 11, claim 12 or claim 13 characterised in that A is sodium, lithium or potassium and where A is sodium, a is preferably in the range of 0.1 to 3.3.

**15.** A composition as claimed in any one of the preceding claims further including at least one of a halogen component, a chalcogen component and a phosphorus component.

**16.** A method for the conversion of methane to produce hydrocarbons having a greater number of carbon atoms, said method comprising contacting methane with a composition as claimed in any one of claims 1 to 15 under conditions to form said hydrocarbons, by-product water and a reduced form of said at least one compound.

**17.** A method for the dehydrogenation of dehydrogenatable hydrocarbon, said method comprising contacting a gas comprising dehydrogenatable hydrocarbon with a composition as claimed in any one of claims 1 to 15 to produce dehydrogenataed hydrocarbon, water and a reduced form of said at least one compound.

**Patentansprüche**

**1.** Katalysator enthaltend (a) mindestens eine Verbindung, ausgewählt aus den Oxiden und sauerstoffhaltigen Verbindungen von mindestens einem Metall, wobei die Verbindung bei Kontakt mit Methan bei einer Temperatur im Bereich von 500° bis 1000°C reduzierbar ist, um höhere Kohlenwasserstoffe und Wasser zu erzeugen und (b) einen Träger, dadurch gekennzeichnet, daß die Zusammensetzung weiterhin (c) mindestens ein Alkalimetall oder eine Verbindung davon umfaßt und der Träger aus mindestens einem von (i) hydroxilierten Magnesia, das vor Zugabe des Materials, das mindestens eine Verbindung aus (a) ergibt gebrannt wurde, und (ii) gemischten Oxiden von Silicium und mindestens einem Erdalkalimetall ausgewählt ist.

2. Zusammensetzung nach Anspruch 1, dadurch gekennzeichnet, daß das hydroxilierte Magnesia von Magnesiumhydroxid oder einer magnesiumhaltigen Komponente, die mit einem hydroxilhaltigen Material in Kontakt gebracht wurde stammt.

3. Zusammensetzung nach Anspruch 1, dadurch gekennzeichnet, daß der Träger Magnesia und Kieselerde in einem Molverhältnis von Magnesia zu Kieselerde von 50 : 1 bis 1 : 1, bevorzugter 30 : 1 bis 5 : 1, enthält.

4. Zusammensetzung nach Anspruch 1 oder 3, dadurch gekennzeichnet, daß das Molverhältnis von der Kieselerde zum Alkalimetall im Bereich von 0,5 : 1 bis 10 : 1 liegt.

5. Zusammensetzung nach einem der Ansprüche 1 bis 4, dadurch gekennzeichnet, daß die Oberfläche mindestens eines Teils des Träger bei einer erhöhten Temperatur vor Zugabe der mindestens einen Verbindung gesintert wird.

6. Zusammensetzung nach Anspruch 5, dadurch gekennzeichnet, daß das Sintern eine Erwärmung des Trägers auf etwa 0,33 der normalen Schmelztemperatur des Materials des Trägers umfaßt.

7. Zusammensetzung nach Anspruch 5 oder 6, dadurch gekennzeichnet, daß die Oberfläche des Trägers von 30 bis 90 $m^2$ pro Gramm reicht.

8. Zusammensetzung nach einem der Ansprüche 1 bis 7, dadurch gekennzeichnet, daß mindestens ein Metall der Verbindung (a) aus Mangan, Zinn, Indium, Germanium, Antimon, Blei, Wismuth und Mischungen davon und das Alkalimetall aus Lithium, Natrium, Kalium, Rubidium, Cäsium und Mischungen davon, vorzugsweise Natrium, ausgewählt ist.

9. Zusammensetzung nach einem der Ansprüche 1 bis 8, dadurch gekennzeichnet, daß mindestens ein Metall der Verbindung (a) Mangan enthält und in der die Menge des vorhandenen Mangans im Bereich von 1 bis 40 Gew.-% liegt und vorzugsweise von 5 bis 30 Gew.-% beträgt, bezogen auf das vereinigte Gewicht von Mangan und Träger, wobei die Zusammensetzung im wesentlichen frei von katalytisch wirksamen Eisen ist.

10. Zusammensetzung nach Anspruch 9, dadurch gekennzeichnet, daß das Atomverhältnis des Alkalimetalls zum Mangan im Bereich von 0,01 : 1 bis 10 : 1 liegt.

11. Zusammensetzung nach Anspruch 9 oder 10, dadurch gekennzeichnet, daß die Zusammensetzung der Formel

$MnA_aB_bSi_cO_x$

entspricht, in der A mindestens ein Alkalimetall bedeutet, B mindestens ein Erdalkalimetall ist, a im Bereich von 0,01 bis 10 liegt, b im Bereich 0,1 bis 90 liegt, c im Bereich von 1 bis 90 liegt, die Summe von b + c > 1 ist und x die Zahl der Sauerstoffatome bedeutet, die durch den Valenzstatus der anderen Elemente erforderlich sind.

12. Zusammensetzung nach Anspruch 11, dadurch gekennzeichnet, daß B Magnesium bedeutet und b vorzugsweise im Bereich von 0,6 bis 10 liegt oder B Calcium bedeutet und b vorzugsweise im Bereich von 0,4 bis 10 liegt oder B Barium bedeutet und b vorzugsweise im Bereich von 0,1 bis 5 liegt.

13. Zusammensetzung nach Anspruch 11 oder 12, dadurch gekennzeichnet, daß B Magnesium ist und das Verhältnis von c zu b im Bereich von 2 : 1 bis 3 : 1 liegt oder das Verhältnis von b zu c im Bereich von 5 : 1 bis 30 : 1 liegt.

14. Zusammensetzung nach Anspruch 11, 12 oder 13, dadurch gekennzeichnet, daß A Natrium, Lithium oder Kalium ist und wenn A Natrium ist, a vorzugsweise im Bereich von 0,1 bis 3,3 liegt.

EP 0 179 869 B1

**15.** Zusammensetzung nach einem der vorhergehenden Ansprüche, die weiterhin mindestens eine Komponente aus einer Halogenkomponente, einer Chalcogenkomponente und einer Phosphorkomponente enthält.

**16.** Verfahren zur Umwandlung von Methan zur Herstellung von Kohlenwasserstoffen mit einer *größeren* Anzahl von kohlenstoffatomen, bei dem man Methan mit einer Zusammensetzung nach einem der Ansprüche 1 bis 15 unter Bedingungen zur Bildung der Kohlenwasserstoffe, des Nebenproduktes Wasser und einer reduzierten Form der mindestens einen Verbindung in Kontakt bringt.

**17.** Verfahren zur Dehydrierung eines dehydrierbaren Kohlenwasserstoffes, bei dem ein Gas, das einen dehydrierbaren Kohlenwasserstoff enthält, mit einer Zusammensetzung nach einem der Ansprüche 1 bis 15 in Kontakt gebracht wird, um einen dehydrierten Kohlenwasserstoffe, Wasser und eine reduzierte Form der mindestens einen Verbindung herzustellen.

**Revendications**

**1.** Catalyseur comprenant (a) au moins un composé, choisi parmi les oxydes et les composés contenant de l'oxygène d'au moins un métal, lequel composé peut être réduit au contact du méthane à une température de l'ordre de 500 à 1000°C pour produire des hydrocarbures supérieurs et de l'eau, et (b) un support, caractérisé en ce que la composition comprend de plus (c) au moins un métal alcalin ou un composé de celui-ci, et que ce support comprend au moins un composé choisi parmi (i) une magnésie hydroxylée qui a été calcinée avant l'addition du matériau fournissant ce composé de (a) au moins présent, et (ii) des oxydes mixtes de silicium et d'au moins un métal alcalino-terreux.

**2.** Composition suivant la revendication 1, caractérisée en ce que la magnésie hydroxylée provient de l'hydroxyde de magnésium ou d'un composant contenant du magnésium mis en contact avec un matériau contenant le groupe hydroxy.

**3.** Composition suivant la revendication 1, caractérisée en ce que ce support comprend de la magnésie et de la silice selon un rapport molaire de magnésie à silice de 50/1 à 1/1, de préférence de 30/1 à 5/1.

**4.** Composition suivant les revendications 1 ou 3, caractérisée en ce que le rapport molaire de cette silice à ce métal alcalin est de l'ordre de 0,5/1 à 10/1.

**5.** Composition suivant l'une quelconque des revendications 1 à 4, caractérisée en ce que la surface d'au moins une partie de ce support est frittée à une température élevée avant l'addition de ce composé au moins présent.

**6.** Composition suivant la revendication 5, caractérisée en ce que ce frittage comprend le chauffage de ce support à environ 0,33 de la température de fusion normale du matériau de ce support.

**7.** Composition suivant les revendications 5 ou 6, caractérisée en ce que l'aire superficielle de ce support est de l'ordre de 30 à 90m$^2$/g.

**8.** Composition suivant l'une quelconque des revendications 1 à 7, caractérisée en ce que ce métal du composé (a) au moins présent est choisi parmi le manganèse, l'étain, l'indium, le germanium, l'antimoine, le plomb, le bismuth et leurs mélanges; et que ce métal alcalin est choisi parmi le lithium, le sodium, le potassium, le rubidium, le césium et leurs mélanges, et est de préférence le sodium.

**9.** Composition suivant l'une quelconque des revendications 1 à 8, caractérisée en ce que ce métal du composé (a) au moins présent est le manganèse et que la quantité de ce manganèse présent est dans la gamme de 1 à 40% en poids et de préférence de 5 à 30% en poids, par rapport au poids combiné de ce manganèse et de ce support, cette composition étant pratiquement dépourvue de fer ayant une activité catalytique.

**10.** Composition suivant la revendication 9, caractérisée en ce que le rapport atomique de ce métal alcalin à ce manganèse est dans la gamme de 0,01/1 à 10/1.

14

**11.** Composition suivant les revendications 9 ou 10, caractérisée en ce que cette composition satisfait à la formule :

$$MnA_aB_bSi_cO_x$$

dans laquelle A est au moins un métal alcalin, B est au moins un métal alcalino-terreux, a est dans la gamme de 0,01 à 10, b est dans la gamme de 0,1 à 90, c est dans la gamme de 1 à 90, la somme de b + c est supérieure à 1 et x est le nombre d'atomes d'oxygène requis par les états de valence des autres éléments.

**12.** Composition suivant la revendication 11, caractérisée en ce que B est le magnésium et b est de préférence dans la gamme de 0,6 à 10, ou B est le calcium et b est de préférence dans la gamme de 0,4 à 10, ou B est le baryum et b est de préférence dans la gamme de 0,1 à 5.

**13.** Composition suivant les revendications 11 ou 12, caractérisée en ce que B est le magnésium et que le rapport c/b est dans la gamme de 2/1 à 3/1 ou que le rapport b/c est dans la gamme de 5/1 à 30/1.

**14.** Composition suivant les revendications 11, 12 ou 13, caractérisée en ce que A est le sodium, le lithium ou le potassium et que, lorsque A est le sodium, a est de préférence dans la gamme de 0,1 à 3,3.

**15.** Composition suivant l'une quelconque des revendications précédentes, caractérisée en ce qu'elle comprend de plus au moins un composant halogène, un composant chalcogène et un composant phosphore.

**16.** Procédé de conversion du méthane pour produire des hydrocarbures ayant un plus grand nombre d'atomes de carbone, comprenant la mise en contact du méthane avec une composition suivant l'une quelconque des revendications 1 à 15 dans des conditions convenables pour former ces hydrocarbures, de l'eau sous-produite et une forme réduite de ce composé au moins présent.

**17.** Procédé pour la déshydrogénation d'un hydrocarbure pouvant être déshydrogéné, comprenant la mise en contact d'un gaz comprenant un hydrocarbure pouvant être déshydrogéné avec une composition suivant l'une quelconque des revendications 1 à 15 pour produire un hydrocarbure déshydrogéné, de l'eau et une forme réduite de ce composé au moins présent.